# EUROPEAN PATENT APPLICATION

(11) **EP 1 241 113 A1**
(43) Date of publication of application: **18.09.2002**
(21) Application number: 01105410.3
(22) Date of filing: 12.03.2001
(51) Int. Cl.: B65D 83/14, A61K 9/00

(54) **Inhaler with means for improving chemical stability of medicinal aerosol solution contained therein**

(71) Applicant: CHIESI FARMACEUTICI S.p.A., 43100 Parma (IT)
(72) Inventor: Lewis, David, 43100 Parma (IT); Ganderton, David, 43100 Parma (IT); Meakin, Brian, 43100 Parma (IT); Brambilla, Gaetano, 43100 Parma (IT); Ferraris, Alessandra, 43100 Parma (IT)
(74) Representative: Albrecht, Thomas, Dr.

(57) **Abstract**

The present invention relates to a medicinal aerosol solution formulation product with improved chemical stability, comprising a pressurized metered dose inhaler (10), comprising an aerosol canister (16) equipped with a metering valve (18) and containing a medicinal aerosol solution formulation (12) containing an active ingredient subject to a degradation by means of peroxides, a hydrofluorocarbon propellant, a co-solvent and optionally a low-volatility component, wherein part or all of the internal surfaces of said inhaler consists of stainless steel, anodized aluminum or are lined with an inert organic coating, and wherein the canister has a rim with rounded edges (40,42,44,46) which avoids breaks in the rubbers used as valve gaskets (30).

## Description

The present invention relates to medicinal aerosol products and, in particular, to aerosol products such as metered dose inhalers for delivery of aerosol solution formulations containing an active ingredient subject to a degradation by means of peroxides. In a preferred embodiment of the invention the active ingredient is a corticosteroid and more preferably a 20-ketosteroid. These kinds of active ingredients have been found to be highly susceptible to chemical degradation when formulated as solution aerosol products. The present invention provides a way of improving chemical stability of such steroids in aerosol solution formulations.

Pressurized metered dose inhalers are well known devices for administering pharmaceutical products to the respiratory tract by inhalation.

Active materials commonly delivered by inhalation include bronchodilators such as β2 agonists and anticholinergics, corticosteroids, anti-leukotrienes, anti-allergics and other materials that may be efficiently administered by inhalation, thus increasing the therapeutic index and reducing side effects of the active material.

MDI uses a propellant to expel droplets containing the pharmaceutical product to the respiratory tract as an aerosol.

For many years the preferred propellants used in aerosols for pharmaceutical use have been a group of chlorofluorocarbons which are commonly called Freons or CFCs, such as CCl₃F (Freon 11 or CFC-11), CCl₂F₂ (Freon 12 or CFC-12), and CClF₂-CClF₂ (Freon 114 or CFC-114).

Recently, the chlorofluorocarbon (CFC) propellants such as Freon 11 and Freon 12 have been implicated in the destruction of the ozone layer and their production is being phased out.

Hydrofluoroalkanes [(HFAs) known also as hydro-fluorocarbons (HFCs)] contain no chlorine and are considered less destructive to ozone and these are proposed as substitutes for CFCs.

HFAs and in particular 1,1,1,2-tetrafluoroethane (HFA 134a) and 1,1,1,2,3,3,3-heptafluoropropane (HFA 227) have been acknowledged to be the best candidates for non-CFC propellants and a number of medicinal aerosol formulations using such HFA propellant systems have been disclosed.

Many of these applications, in which HFAs are used as propellant, propose the addition of one or more of adjuvants including compounds acting as co-solvents, surface active agents including fluorinated and non-fluorinated surfactants, dispersing agents including alkylpolyethoxylates and stabilizers.

Compositions for aerosol administration via MDIs can be solutions or suspensions. The solution type aerosol formulation contains the medicament dissolved or solubilized in the propellant, or a mixture of propellant and co-solvent. The suspension type aerosol formulation contains the medicament in the form of particles which are dispersed in the propellant. The suspension type aerosol formulations usually contain a surfactant, and can also include a co-solvent. Solution compositions offer several advantages: they are convenient to manufacture being the active ingredient substantially completely dissolved in the propellant vehicle and obviate physical stability problems associated with suspension compositions, such as increase of particle size, crystal polymorphism, flocculation, particle aggregation, all of which affect dose uniformity.

On the other hand the widespread use of solution formulations is limited by their chemical instability, causing the formation of degradation products.

The international application WO 00/30608 of the applicant relates to pressurized metered dose inhalers wherein part or all of the internal surfaces consist of stainless steel, anodized aluminum or are lined with an inert organic coating to enhance the chemical stability of active ingredients in solution in a hydrofluorocarbon propellant, a co-solvent and optionally a low volatility component.

WO 96/32099 discloses metered dose inhalers for the administration of different active ingredients in suspension in the propellant, wherein the internal surfaces of the inhaler are partially or completely coated with one or more fluorocarbon polymers optionally in combination with one or more non-fluorocarbon polymers to reduce or essentially eliminate the problem of adhesion or deposition of the active ingredient particles on the can walls and thus ensure consistent delivery of the aerosol from the MDI.

It is also known from Eur. J. Pharm. Biopharm. 1997, 44, 195 that suspensions of drugs in HFA propellant are frequently subjected to absorption of the drug particles on the valves and on the internal walls of the inhaler.

WO 95/17195 describes aerosol compositions comprising flunisolide, ethanol and HFA propellants. It is stated in the document that conventional aerosol canisters can be used to contain the composition and that certain containers enhance its chemical and physical stability. It is suggested that the composition can be preferably contained in vials coated with resins such as epoxy resins (e.g. epoxy-phenolic resins and epoxy-urea-formaldehyde resins).

The compositions are preferably dispensed via a valve assembly wherein the diaphragm is fashioned by extrusion, injection molding or compression molding from a thermoplastic material such as FLEXOMER™ GERS 1085 NT polyolefin (Union Carbide). Another suitable valve rubber is a nitrile rubber ("DB-218") available from American Gasket and Rubber, Schiller Park. Illinois.

WO 00/35458 affords the problem of preparing a stable solution formulation of budesonide, suitable for use as an aerosol, that remains chemically and physically stable during storage at ambient conditions of temperature and humidity.

The above objectives are achieved with a formulation containing unusual high concentrations of the co-solvent. The most preferred co-solvent is ethanol and it is preferably present in an amount of at least 10% by weight, more preferably at least 15% by weight, even more preferably at least 20% by weight and most preferably at least 25% by weight.

On the other hand it is well known that high quantities of ethanol are detrimental to the formulation performance, since they cause a decrease of the fine particle dose, i.e. of the quantity of particles of the active ingredient reaching the peripheral airways.

WO 00/78286 teaches that certain steroids, particularly 20-ketosteroids, are highly susceptible to chemical degradation when formulated as solution aerosol products and stored in contact with aerosol containers made of metal, usually aluminum. The chemical degradation is particularly influenced by the metal oxide e.g. Al₂O₃ layer that forms on the interior surface of the container.

In WO 00/78286 the chemical instability of aerosol formulations containing steroids is emphasized. A great deal of research has been directed at steroid degradation. Chemical degradation is especially problematic when the steroid is dissolved in the formulation and, consequently, the vast majority of marketed MDI steroid products are formulated as particulate suspensions of the steroid, which are much less susceptible to chemical degradation than solutions. The inventors according to WO 00/78286 believe that all currently marketed CFC-containing MDI products for delivering steroids are available only as particulate suspension formulations in CFC-propellants. However, suspension formulations of a medicament are more likely to encounter problems with physical instability (e.g. agglomeration, crystal growth and deposition on the container wall, all resulting in inconsistent dosage delivery).

Furthermore, according to WO 00/78286, until now there has been no way to identify which steroids are likely to be most stable as solution aerosols and which will be most sensitive to degradation in solution aerosol products or how to reduce such degradation.

The suggested solution in WO 00/78286 is to use an aerosol container having a non-metal interior surface. It is also suggested to similarly coat the metal valve components in contact with the formulation with an inert material. The inert material is selected from epoxy-phenolic lacquer, perfluoroethylenepropylene and a very thin layer of fused silica glass.

Alumina-catalyzed degradation of corticosteroids such as budesonide or triamcinolone acetonide in ethanol solution were also presented to the American Association of Pharmaceutical Scientists - AAPS meeting held in Indianapolis , IN (USA) from October 20 to November 2, 2000.

It has now been found that the chemical degradation of steroids in solution in a HFA propellant/ethanol system can depend not only upon metal oxides of the layer of the interior surface of the container but also upon the peroxides released from the rubbers used as valve gasket.

Pressurized metered dose inhalers are known devices, usually consisting of a main body or canister (can) acting as a reservoir for the aerosol formulation, a cap sealing the main body and a metering valve fitted in the cap.

MDIs are usually made of a conventional material such as aluminum, tin plate, glass, plastic and the like.

The filling of a metered dose inhaler with a composition comprises the following steps:
1. Weighing the required components ( one or more active materials, one or more co-solvents, optional excipients) into a canister;
2. Crimping a valve upon the canister;
3. Adding a known amount of propellant through the valve into the canister.

It has now been found that the crimping of the valve upon the can may cause breaks in the rubbers used as valve gaskets and consequent release of peroxides with time.

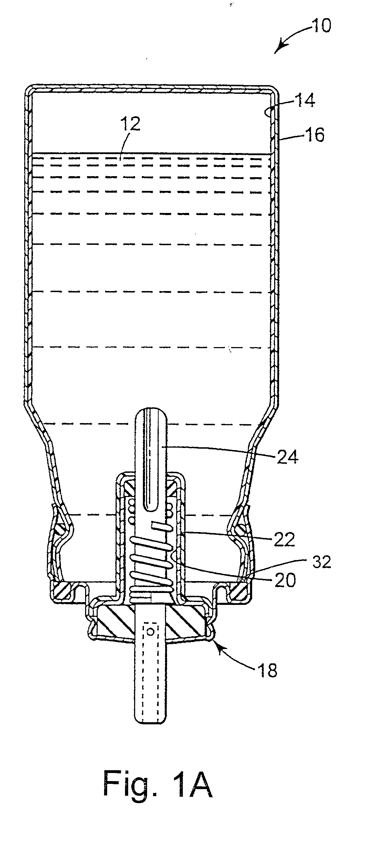

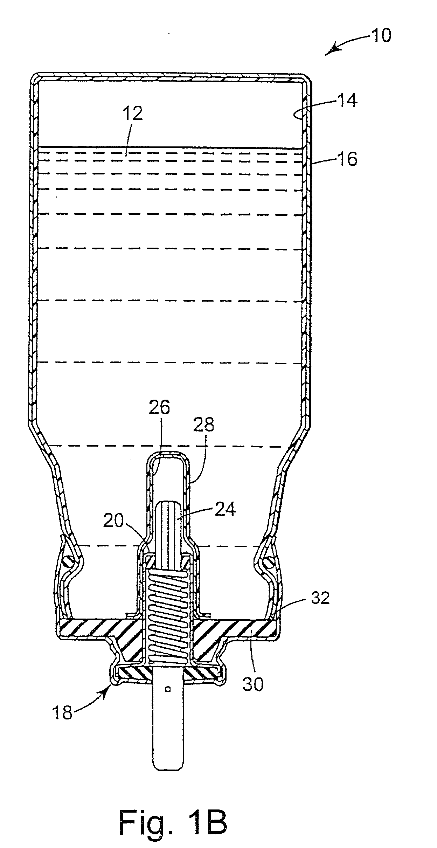

The standard canisters, in fact, have a cutting edge opening; see Figures 1A and 1B. Figures 1A and 1B show a cross-sectional view of a metered dose inhaler containing a medicinal 20-ketosteroid formulation according to the prior art (WO 00/78286).

The present invention provides a medicinal aerosol solution formulation product with enhanced chemical stability. Such a product consists of a main body or canister (can) acting as a reservoir for the aerosol formulation, a cap sealing the main body and a metering valve fitted in the cap, characterized in that the canister has no cutting edge opening, i.e. it has rounded edges. Moreover, preferably the valves are washed with a suitable pharmaceutically acceptable solvent, preferably ethanol before the metered dose inhaler is built. In general, solvents which are pharmaceutically acceptable and endowed with adequate capacity of extraction of oxides and peroxides can be utilized. In the medicinal aerosol solution formulation product of the present invention breaks in the rubbers used as valve gaskets are excluded by avoiding a cutting edge opening at the rim of the canisters.

According to the present invention, a canister having a rim with rounded edges, preferably a rolled neck, or a rolled in rim, a part or full rollover rim is used for the preparation of aerosol solution formulation products containing an active ingredient subject to a degradation by means of peroxides.

In a preferred embodiment of the invention the active ingredient is a corticosteroid and more preferably a 20-ketosteroid.

Preferably the pressurized metered dose inhaler (MDI) used in the present invention is one as described in applicant's earlier patent application PCT/EP99/09002 published as WO 00/30608. Accordingly, in a preferred embodiment of the present invention, part or all of the internal surfaces of the MDI consist of stainless steel, anodized aluminum or is lined with an inert organic coating. Any kind of stainless steel may be used. The preferred material of the aerosol cans is anodized aluminum. One of the preferred coatings exists of epoxy-phenol resin. Suitable epoxy-phenol resins are commercially available. Other examples for preferred inert organic coatings are perfluoroalkoxyalkane, perfluoroalkoxyalkylene, perfluoroalkylenes such as polytetrafluoroethylene, fluorinated-ethylene-propylene, polyether sulfone and a copolymer fluorinated-ethylene-propylene polyether sulfone.

Other suitable coatings could be polyamide, polyimide, polyamideimide, polyphenylene sulfide or their combinations.

The invention will now be described with reference to the following drawings. Figures 1A and 1B show a metered dose inhaler containing a medicinal 20-ketosteroid formulation according to the prior art (WO 00/78286). Figure 1B is the same as Figure 1A but with a modified valve configuration.

Figure 1A shows a medicinal aerosol device 10 comprising a pressurizable metal aerosol container 16 equipped with a metering valve 18. The metal container 16 is preferably made of aluminum (i.e. aluminum or aluminum alloy) and has an inert interior coating layer 14. The metering valve 18 includes a metal metering chamber 20 with a coating layer 22.

Figure 1B shows an alternative embodiment that is essentially the same as Figure 1A but utilizes a fixed bottle emptier 26 with coating layer 28. Also, a solution gasket 30 is used to further prevent contact of the formulation with metal components.

WO 00/78286 does not appreciate to avoid any breakage of the rubbers used as valve gaskets to avoid any release of peroxides with time. As can be taken from Figures 1A and 1B, the canisters used according to WO 00/78286 have a cutting edge opening 32 in contact with the rubbers used as valve gaskets which may cause breaks in the rubbers.

Instead of a cutting edge 32 the canisters according to the present invention have a rounded edge in order to avoid any breaks in the rubbers used as valve gaskets. The rim can be of any kind avoiding contact of a sharp edge with the rubbers used as valve gaskets.

Figure 2 is a cross-sectional view of a canister with a rolled neck 40 used in a metered dose inhaler containing a medicinal aerosol solution formulation with enhanced chemical stability according to the present invention.

Figure 3 is a cross-sectional view of the neck of a canister with a rolled-in rim 42.

Figure 4 is a cross-sectional view of the neck of a canister with a part rollover rim 44.

Figure 5 is a cross-sectional view of the neck of a canister with a full rollover rim 46.

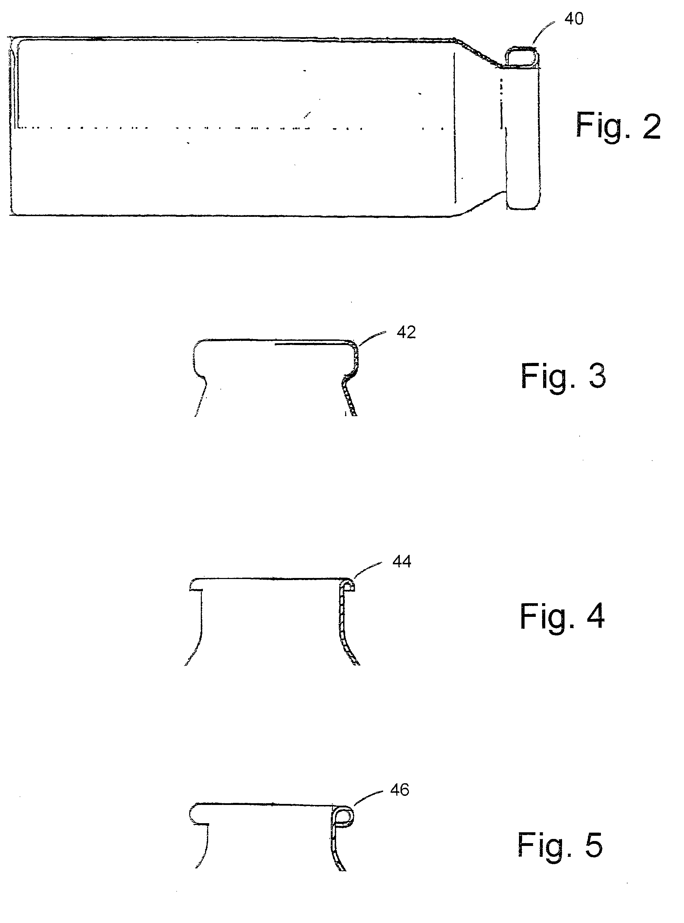

The canisters according to Figures 2 to 5 can be used in conventional metered dose inhalers or in metered dose inhalers as described in applicant's earlier patent application WO 00/30608 wherein part or all of the internal surfaces consist of stainless steel, anodized aluminum or are lined with an inert organic coating.

Moreover, in a particular preferred embodiment the valve gaskets are washed before crimping of the valve upon the can with ethanol in order to remove any impurities such as metal oxides and peroxides from the rubbers used as valve gaskets.

The examples which follow have been conducted with a solution formulation of budesonide in ethanol, glycerol and HFA 134a.

The invention naturally applies to any formulation of a steroid and especially of a 20-ketosteroid in solution in a HFA propellant which meets with problems of chemical stability.

Examples of this kind of steroids are budesonide and its epimers, mometasone furoate, triamcinolone acetonide, butixocort, ciclesonide.

It has been observed in budesonide solution formulations in HFA 134a (norflurane) and ethanol put on stability at various temperatures both up-right and inverted that notwithstanding the use of anodized aluminum or epoxy-phenol lacquered cans at high temperature the formulations are chemically stable for the first months, after which an acceleration of the oxidation process takes place.

The chemical degradation is slowed when valves extracted (washed) with ethanol are employed. In fact the washing removes the impurities and among these also peroxides and possible metal oxides surrounding the metallic part of the valve.

### EXAMPLE 1

Cans of Budesonide in solution according to the following formulation were manufactured:

| | |
|---|---|
| Budesonide | 400 mg (200 mcg/shot) |
| Glycerol | 1.3% (w/w) |
| Ethanol | 15.0% (w/w) |
| Norflurane | q.b. a 100 ml |

Anodized and lacquered cans and non ethanol extracted valves were utilized. The cans were put on stability at 50°C (inverted)

A high degree of degradation was observed.

**Table 1**

| Stability of Budesonide solution formulations (inverted) Recovery (%) of budesonide after storage at different times and temperatures (average values referred to two tests) | | | |
|---|---|---|---|
| Can Type (cut edge can) | t = 0 | t = 2 months T = 50°C | t = 5 months T = 50°C |
| | | | |
| Anodized Aluminum | 99.42 % | - | 33.38 % |
| Lacquered | 99.66 % | - | 36.86 % |
| t = time | | | |
| T = temperature | | | |

The degradation is an oxidative one and it was explained with the presence of peroxides in the rubbers.

### EXAMPLE 2

Cans with the same formulation were prepared and in this case ethanol extracted valves were utilized. During the ethanol extraction the surface of the valves was washed of the peroxides and the solution formulation exhibits a much higher stability.

**Table 2**

| Stability of Budesonide solution formulations (inverted) Recovery (%) of budesonide after storage at different times and temperatures (average values referred to two tests) | | | |
|---|---|---|---|
| Can Type (cut edge can) | t = 0 | t = 2 months T = 50°C | t = 5 months T = 50°C |
| | | | |
| Anodized Aluminum | 99.18 % | 96.64 % | 76.94 % |
| Lacquered Epoxy-Phenol | 99.52 % | 97.28 % | 71.03 % |
| t = time | | | |
| T = temperature | | | |

### Example 3

Two cans of anodized aluminum prepared as per example 2 were submitted to the determination of degradation products of the active ingredient.

It was noticed in this way that after a lag time of 6 months at 40°C the degradation rate increases with the same pattern seen in the first example. The phenomenon does not occur if the cans are stored upright, because a direct contact between the solution and the rubbers is avoided. An explanation of the process is that peroxides may leach into the solution after a while, thus starting a radicalic degradation of Budesonide.

**Table 3**

| Stability of Budesonide solution formulations (anodized aluminum cut edge can) Recovery (%) of degradation products desonide, budesonide-21-aldehyde, budesonide-17-acid (related substances) at different times, temperatures and relative humidities (RH) | | | | | |
|---|---|---|---|---|---|
| TEST | | START | 6months 40°C/75%RH Inverted | 6months 40°C/75%RH Upright | 6months 30°C/60%RH Inverted |
| Related substances (%) : | | | | | |
| -desonide | | 0.15 | 0.14 | 0.17 | <0.06 |
| -bud-21-ald. | | 0.16 | 2.96 | 1.19 | 0.98 |
| -bud-17-ac. | | - | 0.99 | 0.35 | 0.34 |
| -unknowns | | 0.27 | 0.49 | 0.37 | 0.18 |
| TOTAL | | 0.58 | 4.58 | 2.08 | 1.50 |

### EXAMPLE 4

It has been observed that when the experiment is repeated changing only the finishing of the cans, (from cut edge to rolled neck; see Figure 2) the stability of the solution is greatly increased due to two factors:
- the different finishing of the can doesn't cause surface damages and/or cuts of the rubber gaskets, therefore avoiding the exposure of fresh surfaces not exposed during the ethanol extraction process;
- the total gasket area exposed to the solution is much lower.

In conclusion, the combined action of ethanol extraction of the rubber gaskets of the valves and the finishing of the neck of the cans greatly improves the stability of solution formulations that otherwise may incur oxidative degradation triggered by the presence of peroxides in the above mentioned gaskets.

**Table 4**

| Stability of Budesonide solution formulations (Epoxy-Phenol lacquered rolled neck can) Recovery (%) of degradation products desonide, budesonide-21-aldehyde, budesonide-17-acid (related substances) at different times, temperatures and relative humidities (RH) | | | | |
|---|---|---|---|---|
| TEST | | START | 6months 40°C/75%RH Inverted | 12months 25°C/60% RH Inverted |
| Related substances (%) : | | | | |
| -desonide | | 0.14 | 0.23 | 0.16 |
| -bud-21-ald. | | 0.24 | 1.22 | 0.85 |
| -bud-17-ac. | | - | - | - |
| -unknowns | | 0.29 | 0.60 | 0.37 |
| TOTAL | | 0.67 | 2.05 | 1.38 |

## Claims

1. A medicinal aerosol solution formulation product with improved chemical stability, comprising a pressurized metered dose inhaler, comprising
an aerosol canister equipped with a metering valve and containing a medicinal aerosol solution formulation containing an active ingredient subject to a degradation by means of peroxides, a hydrofluorocarbon propellant, a co-solvent and optionally a low-volatility component, wherein part or all of the internal surfaces of said inhaler consists of stainless steel, anodized aluminum or are lined with an inert organic coating, and
wherein
the canister has a rim with rounded edges which avoids breaks in the rubbers used as valve gaskets.

2. Product of claim 1, wherein the canister has a rolled neck.

3. Product of claim 1, wherein the canister has a rolled-in rim.

4. Product according to claim 1, wherein the canister has a part rollover rim.

5. Product according to claim 1, wherein the canister has a full rollover rim.

6. Product according to any one of claims 1 to 5, wherein the valve is washed before crimping of the valve upon the canister with a pharmaceutically acceptable solvent, preferably ethanol.

7. Product according to any one of claims 1 to 6, wherein the active ingredient is a corticosteroid.

8. Product according to claim 7, wherein the corticosteroid is a 20-ketosteroid.

9. Product according to claim 8, wherein the 20-ketosteroid is budesonide and its epimers, mometasone furoate, triamcinolone acetonide, butixocort or ciclesonide.

10. Product according to any one of claims 1 to 9, wherein the low-volatility component is selected from the group consisting of glycerol, propylene glycol, polyethylene glycol and isopropyl myristate.

11. Product according to any one of claims 1 to 10, wherein the co-solvent is ethanol.

12. Product according to any one of claims 1 to 11, wherein the propellant is selected from HFA 227, HFA 134a and their mixtures.

13. Product according to any one of claims 1 to 12, wherein the inert organic coating is perfluoroalkoxyalkane, perfluoroalkoxyalkylene, perfluoroalkylenes such as polytetrafluoroethylene, epoxy-phenol resin or fluorinated-ethylene-propylene, polyether sulfone, or their combinations.

14. Product according to any one of claims 1 to 13, wherein part or all of the internal surfaces are coated with an epoxy-phenol resin.

15. Product according to any one of claims 1 to 14, wherein part or all of the internal surfaces consist of anodized aluminum.

16. Process for making a chemically stable aerosol solution formulation product containing an active ingredient subject to a degradation by means of peroxides by filling into a canister of a pressurized metered dose inhaler an aerosol solution formulation, comprising an active ingredient subject to a degradation by means of peroxides, wherein part or all of the internal surfaces of said inhaler consists of stainless steel, anodized aluminum or are lined with an inert organic coating and wherein the canister has a rim with rounded edges.

17. Process according to claim 16, wherein the canister has a rolled neck, a rolled-in rim, a part rollover rim or a full rollover rim.

18. Process according to claim 16 or 17, wherein the valve is washed with ethanol before crimping of the valve upon the canister.

19. Process for the stabilization of an aerosol solution formulation containing an active ingredient subject to a degradation by means of peroxides contained in a pressurized metered dose inhaler by use of a canister with a rim with rounded edges and by use of a valve which has been washed with ethanol beforehand.

20. Process according to claim 19, wherein a canister is used having a rolled neck.

21. Process according to claim 19, wherein a canister is used with a rolled-in rim, part rollover rim or full rollover rim.
